## Europäisches Patentamt
## European Patent Office
## Office européen des brevets

(19) ㊆

(11) Numéro de publication: **0 063 973**
**B2**

(12) # NOUVEAU FASCICULE DE BREVET EUROPÉEN

(45) Date de publication du nouveau fascicule du brevet:
**20.05.87**

(21) Numéro de dépôt: **82400587.0**

(22) Date de dépôt: **31.03.82**

(51) Int. Cl.⁴: **A 61 K 31/725,** A 61 K 9/06,
C 08 B 37/08

(54) Composition pour collyre à base de sulfate de chondroitine A.

(30) Priorité: **09.04.81 FR 8107106**

(43) Date de publication de la demande:
**03.11.82 Bulletin 82/44**

(45) Mention de la délivrance du brevet:
**14.03.84 Bulletin 84/11**

(45) Mention de la decision concernant l'opposition:
**20.05.87 Bulletin 87/21**

(84) Etats contractants désignés:
**AT BE CH DE GB IT LI LU NL SE**

(73) Titulaire: **Laboratoires POS, F-68240 Kaysersberg (FR)**

(72) Inventeur: **Andermann, Guy, 5, rue des Jonquilles, F-68000 Colmar (FR)**
Inventeur: **Andermann, Claudine, 5, rue des Jonquilles, F-68000 Colmar (FR)**

(74) Mandataire: **Cuer, André, CABINET CUER 30, rue de Léningrad, F-75008 Paris (FR)**

(56) Documents cité:
US-A-3 371 012
US-A-3 547 904

CHEMICAL ABSTRACTS, vol. 84, no. 10, 8 mars 1976, page 393, résumé no. 65256s, Columbus, Ohio (US)
CHEMICAL ABSTRACTS, vol. 82, no. 17, 28 avril 1975, page 95, résumé no. 106992h, Columbus, Ohio (US) A.G. TRAVKIN: "Comparative study of the effect of alpha-tocopherol and chondroitin sulfate A on the retention of viability of corneal tissue from various preserved whole eyeballs under various conditions"
CHEMICAL ABSTRACTS, vol. 74, no. 12, 22 mars 1971, page 235, résumé no. 57316q, Columbus, Ohio (US)
CHEMICAL ABSTRACTS, vol. 96, no. 16, 19 avril 1982, page 461, résumé no. 129739j, Columbus, Ohio (US), S. IWATA et al.: "Studies on

(56) Documents cité: (suite)
biocompatability of contact lens materials. III. The effects of viscous agents on cell adhesion to lens materials"
Vestnik Oftalmologii (1974) (5) p. 61-62
Acta Societatis Ophtalmological 68 (2) p. 64-154 (1964)
Rote Liste 1980, 05105
Monatsblätter für Augenheilkunde (1961) 139 Band, 664

Le dossier contient des informations techniques présentées postérieurement au dépôt de la demande et ne figurant pas dans le présent fascicule.

EP 0 063 973 B2

**0 063 973**

## Description

La présente invention a trait au domaine des compositions pour collyres destinés au traitement des maladies de l'oeil. Elle concerne tout particulièrement un nouveau collyre apte à se substituer à une sécrétion lacrymale anormale ou insuffisante.

On sait que le liquide lacrymal, qui baigne en permanence la cornée, la conjonctive et les culs-de-sac conjonctivaux, joue un important rôle de défense contre les infections et participe à la physiologie de la cornée par le mouvement d'eau et le transport d'oxygène. La sécrétion lacrymale normale-composée d'une sécrétion de base produite par les glandes appropriée et d'une sécrétion réflexe causée par stimulation sensorielle-doit transmettre parfaitement les rayons lumineux du spectre visible et joue un rôle majeur dans la perfection du dioptre air-cornée.

Les affections de sécheresse oculaire, caractérisées par un manque ou une inefficacité des larmes - comme par exemple les syndromes de Gougerot-Sjögren et de Stevens-Johnson - peuvent fréquemment conduire à de graves complications cornéennes avec ulcères à répétitions et/ou des cicatrices et adhérences entre les paupières et le globe de l'oeil, lesquelles aboutissent parfois à la cécité.

On a déjà préconisé, pour le traitement de telles affections oculaires, l'utilisation de médicaments pouvant contenir divers ingrédients actifs tels que par exemple des antipaludiens de synthèse, la bromhexine, l'anétholtrithione par voie orale, l'alcool polyvinylique, des dérivés de la cellulose, l'acétylcystéine par voie locale... etc.

Toutefois, ces médicaments n'ont pas donné satisfaction. Les produits administrés par voie orale ont du être abandonnés du fait de leur toxicité. Par ailleurs, les compositions à action locale ne comblent pas suffisamment les fractions manquantes du film lacrymal et ne présentent pas la tension superficielle souhaitée aux doses adéquates.

Il a maintenant été trouvé que l'on pouvait pallier les inconvénients susvisés et mettre à la disposition des malades un nouveau collyre de grande efficacité, bien toléré et non toxique, capable de traiter les hypolacrymies dues à une insuffisance de sécrétions lacrymales de base, en particulier dans: les kératoconjonctivites sèches, les brûlures oculaires, les larmoiements chroniques des vieillards, les irritations oculaires dues au port de lentilles de contact, et d'autres maladies telles que: syndromes de Gougerot-Sjögren, Riley-Day, maladie de Stevens-Johnson, pemphigus... etc.

Conformément à l'invention, la nouvelle composition pour collyre renferme comme seul constituant actif le sel disodique du sulfate de chondroïtine A.

On sait que le sulfate de chondroïtine est un mucopolysaccharide de haute viscosité existant sous forme endogène dans le sang et l'urine des mammifères et de propriétés pharmacologiques et thérapeutiques souvent comparables à celles de l'héparine. En pratique, il existe plusieurs formes de sulfates de chondroïtine à savoir les sels de type A (ou athéroitine), de type B (ou béta-héparine) et de type C (Merck Index 9ème édition, 1976).

Dans les compositions pharmaceutiques divulguées à ce jour et qui font appel à la chondroïtine comme principe actif, ce sont toujours des mélanges de sulfates et notamment des types A et C precités qui sont mis en oeuvre. C'est le cas par exemple des médicaments à action hypolipémique connus sous les marques Arteparon, Eleparon ou encore des produits, également présentés sous forme de gélules, proposés dans les états de déminéralisation osseuse (marque déposée Structum).

Contrairement à cet art antérieur, l'invention fait appel seul au sulfate pur de type A de la chondroïtine, produit chimique connu en soi se présentant sous la forme d'une poudre blanche hygroscopique, sans saveur ni odeur, soluble dans l'eau et insoluble dans l'alcool, l'acétone, le chloroforme, de poids moléculaire moyen compris entre 25 000 et 50 000. En outre, les indications thérapeutiques découvertes par la Demanderesse pour ce produit, ainsi que les modes de présentation (collyre au lieu de gélules) et les voies d'administration sont totalement différents de ceux préconisés jusqu'ici pour les mélanges de sels précités.

Outre, le constituant actif susvisé, les compositions pour collyres selon l'invention renferment divers agents jouant essentiellement les rôles de conservateurs, agents tampons, isotonisants... de façon connue en soi. Parmi les produits de fonction tampon on peut citer notamment: le phosphate monopotassique et/ou le phosphate dipotassique, le chorure de sodium, des agents complexants comme l'acide éthylène diaminotétracétique; etc. Les produits conservateurs peuvent être constitués par des sels de mercure non toxiques comme par exemple l'acétate ou le borate phénylmercurique, le thimerosal, le gluconate de chlorhexidine, le chlorbutol.. L'agent isotonique peut être choisi également parmi des produits bien connus pour cette fonction; à titre d'exemple, l'acide borique convient particuliérement bien dans ce cas. Bien entendu, comme dans le cas des autres collyres, les compositions sont diluées par de l'eau distillée.

Les quantités de sel disodique sulfate A de chondroïtine mises en oeuvre dans les collyres selon l'invention peuvent varier entre d'assez larges limites, généralement entre 10 et 100 g/litre de collyre. Elles sont toutefois avantageusement maintenues entre les fourchettes de 26 et 40 g/litre. Quant aux proportions des adjuvants annexes précités, elles ne sont évidemment pas critiques et sont fonction du type de produit utilisé. Un exemple de composition sera illustré au cours de la description suivante.

Les nombreuses études de stabilité entreprises par la Demanderesse ont démontré, qu'après une durée de plus de 36 mois, le principe actif conservait sa teneur inchangée et qu'en outre il n'y avait pas interaction entre celui-ci et des récipients en matière plastique (par exemple en polyéthylène) utilses pour la conservation des collyres à base de sulfate A de chondroïtine.

2

# 0 063 973

Des séries d'expériences et abservations faites à partir de compositions de collyres selon l'invention dans les domaines: toxicologie, pharmacologie, pharmacocinétique et biodisponibilité, ont donné des résultats extrémement intéressants.

Au plan de la toxicité, les expériences d'administration per os et par voie intrapéritonéale à des souris et à des rats ont montré une absence totale de toxicité aigue et une dose léthale DL 50 nettement supérieure aux doses maximales d'administration; l'indice d'irritation oculaire est nul chez le lapin et il n'y a pas de toxicité subaiguë chez le rat; les expériences sur chiens ont conclu à une mortalité nulle et à un comportement tout à fait normal (croissance, digestion, aucune lésion constatée après tous les examens pratiqués); aucun effet tératogène n'a été relevé après essais sur lapins et l'on a pu conclure à unetrés bonne tolérance dans l'oeil de l'homme.

Dans le domaine pharmacologique, les résultats d'expérimentations chez le lapin ont été hautement significatifs et ont montré, après simulation des yeux secs et traitement au collyre que l'on obtenait une excellente humidification de l'oeil sec. Les essais sur bovins ont permis de conclure à une très mauvaise pénétration du collyre dans l'oeil, avec maintien de l'intégrité de la cornée. L'étude de la biodisponibilité de la molécule des sulfate de chondroïtine A, après administration orale chez le lapin, n'a pas permis de mettre en évidence l'absorption orale de cette substance; l'absence de biodisponibilité est vraisemblablement due au poids moléculaire très élevé du principe actif mis en oeuvre selon l'invention; aucune toxicité systémique n'a pu, en tout cas, être mis en évidence.

Enfin, les séries de nombreuses études cliniques sur malades, entreprises en divers centres hospitaliers par des experts cliniciens en ophtalmologie ont mis en évidence la grande efficacité du médicament, nettement supérieure à celle de collyres de références dont le sérum physiologique à 9‰ de Nacl, connu pour être le meilleur succédané des larmes.

L'invention sera mieux comprise par une description détaillée des essais et observations entrepris à partir d'un exemple, de réalisation d'une composition de collyre selon l'invention.

## Exemple de réalisation

Le collyre retenu pour les séries d'expérimentations avait la composition pondérale suivante:

| | |
|---|---|
| Sel disodique du sulfate de chondroïtine A | 3g |
| Phosphate dipotassique anhydre | 0,25 g |
| Phosphate monopotassique anhydre | 0,113 g |
| Acideborique | 1,2 g |
| Borate phénylmercurique | $\leqslant 0,0033$ g |
| Eau purifiée, quantité suffisante pour | 100 ml |

Le sel disodique du principe actif avait une pureté d'au moins 95% et un poids moléculaire moyen de l'ordre de 30 000.

### a) Essai d'innocuité du collyre

Les observations ont été faites pendant 2 semaines après injection par sondage per os de 100 mg de principe actif/kg sur 12 lapins albinos New Zealand de poids moyen 3 kgs et de 280 mg de principe actif/kg sur 24 rats de poids moyen 210 g.

Aucune mortalité n'a été constatée et on a observé aucune anomalie ni au niveau du comportement des animaux ni au niveau du tractus gastro-intestinal après autopsie.

### b) Recherche de la dose léthale 50 (DL 50) chez la souris et le rat

Les études ont été menées simultanément d'une part sur des lots de 50 souris mâles et 50 souris femelles et, d'autre part, sur un lot de 100 rats également répartis entre mâles et femelles. Après 8 jours d'acclimation, on a effectué des injections de collyre par voie intrapéritonéale et effectué des axamens cliniques continus pendant les six premières heures puis, à intervalles réguliers, pendant les 13 jours suivant l'injection.

L'injection de 0,5 ml de collyre correspondait sensiblement à l'injection péritonéale de 3,13-4,70-7,10-10,65 ou 16,0 g/kg du principe actif. La DL 50 approchée était de l'ordre de 12,8 g/kg pour les souris mâles avec des limites de 9,7 et 16,9 g/kg et de 13,0 g/kg pour les souris femelles avec des limites de 8,7 à 19,4 g/kg. Du fait que la posologie moyenne du collyre est de 1 à 2 gouttes par oeil soit 1,5 à 3 mg de principe actif, on voit que, en comparaison des doses de DL 50, le collyre selon l'invention ne présente aucune toxicité.

Les résultats étaient du même type dans le cas des rats avec des DL 50 moyennes de 8,10 g/kg pour les rats mâles et 8,30 g/kg pour les rats femelles.

Dans des expériences correspondant à une administration par voie orale du principe actif chez des souris et par voie-sous-cutanée à des rats, on a pu conclure que, dans le premier cas, la DL 50 était nettement supérieure à la dose maximale administrée (soit > 22 g/kg) et, dans le second cas la DL 50 était supérieure à 10 g/kg.

#### c) Toxicité subaiguë

On a effectué des expérimentations sur 6 lapins Albinos répartis à égalité de sexe en instillant le collyre précité dans la paupière inférieure éversée de l'oeil droit, l'oeil gauche servant de témoin pour chaque lapin. Les animaux ont reçu 4 fois 4 gouttes par jour, à intervalles de 2 heures. Le traitement a été effectué pendant 30 jours à raison de 6 jours par semaine. Les examens des yeux étaient entrepris chaque semaine dans l'ordre suivant: conjonctive, iris, cornée. Il s'est avéré que l'indice d'irritation oculaire (1.1.0) était nul; le produit avait donc un pouvoir irritant nul.

D'autres séries d'essais ont été entrepris par voie orale sur des rats en administrant, à raison de 7 jours par semaine pendant 8 semaines, 100 mg/kg per os de principe actif. Les examens pratiqués régulièrement sur les animaux ont permis de conclure que: l'état clinique n'était pas modifié, la croissance était régulière, il n'y avait pas le signe d'intoxication sanguine ni modification de l'aspect et du poids des viscères. Ceci a permis de conclure à la toxicité nulle du principe actif selon l'invention.

#### d) toxicité chronique

Les essais ont été entrepris sur une série de 22 chiens de race Beagle répartis en trois groupes:

. le premier (8 chiens) recevait 60 mg/kg/jour de sel disodique de sulfate de chondroïtine A; ceci en une seule prise par voie buccale, 6 jours par semaine.

. le secon (8 chiens) recevait 200 mg/kg/jour du même principe actif dans les mêmes conditions que ci-dessus.

. le troisième (6 chiens) servait de lot témoin.

Pendant toute la durée du traitement, on a noté l'aspect et le comportement des animaux. Après 3 mois de traitement, on a sacrifié les chiens puis procédé à un examen hématologique complet et à une étude analytique des divers organes (foie, rein, rate, coeur, poumon, estomac, intestin, surrénale, pancréas, oeil).

On a constaté que la mortalité était nulle pour tous les groupes d'animaux (traités ou non). Le comportement des animaux était normal avec croissance pondérale sans trouble digestif. Les examens hématologiques ne montraient pas de changement significatif du au traitement. Par ailleurs, les tests anatomopathologiques, macroscopiques et histologiques n'ont mis en évidence aucune lésion.

On a donc pu conclure, à la lumière de ces résultats, que le principe actif du collyre selon l'invention pouvait être administré à l'homme aux doses de 1 g/jour au cours des essais cliniques, cette dose étant bien supérieure à celle recommandée pour l'administration quotidienne chez l'homme de la composition de collyre elle-même.

#### e) toxicité foetale

On a effectué des expertises sur des lots e 60 lapins et 60 rats (répartis par sexe) avec des doses de principe actif en gélules de 0,250 g. Après avoir sacrifié le 28ème jour de gestation la moitié des animaux femelles on n'a pu mettre en évidence aucun effet tératogène du principe actif selon l'invention.

#### f) tolérance locale

L'évaluation de cette toxicité a été faite par mesure de paramètres physiologiques tels que la différence de potentiel existant entre la face épithéliale et la face endothéliale de la cornée ou encore l'épaisseur de cette cornée. Au contact de produits toxiques, on constate en effet un épaississement de la cornée et une chute de d.d.p. transcornéen.

Les expériences, qui portaient à la fois sur le collyre de l'exemple précité, sur l'excipient seul (sans principe actif) de ce collyre et sur des produits connus tels que la tétracaine, et le benoxinate et un ammonium quaternaire, ont montré que ces produits étaient toxiques pour la cornée alors qu'aucune toxicité du collyre selon l'invention n'était décelable in vitro, même après 6 heures d'expérience.

Cela a permis de conclure que le collyre conforme à l'invention pouvait être administré de manière chronique chez l'homme sans lésion des structures épithéliales de la cornée.

#### g) Etude pharmacodynamique du collyre

Cette étude a été envisagée pour permettre de simuler les "yeux secs" chez le lapin et de traiter efficacement ces "yeux secs", ceci par un examen quantitatif des larmes puis une étude de stabilité du film lacrymal.

Pour cela, on a utilisé le test de SCHIRMER, bien connu pour l'homme, en l'appliquant à des séries de 6 lapins Albinos de 3 kg environ. On avait provoqué auparavant une hypolacrymie expérimentale par instillation d'un collyre à base de bétoxycaine (anesthésique local). On a ensuite étudié le comportement du collyre selon l'invention à l'égard de ce déssèchement.

Les essais ont été entrepris en trois étapes:

. étude quantitative du film lacrymal chez l'animal aux yeux normaux;

. même étude sur l'animal aux yeux normaux, anesthésié

. étude chez l'animal anesthésié, traité au collyre selon l'invention.

En comparant, par calculs statistiques, les valeurs obtenues, exprimées en mm de bandelette de papier filtre humidifié (oeil normal/oeil sec) on a pu constater une différence très significative entre les deux séries de valeurs comparées, puisque l'assèchement de l'oeil était de l'ordre de 45%.

En effectuant ensuite les séries de comparaison pour les résultats d'essais oeiltraité/oeil sec et oeil normal/oeil sec, on a pu mettre en évidence une humidification évidente de l'oeil sec par le collyre de l'invention et une récupération d'au moins 80% de l'humidité normale de l'oeil.

Dans un deuxième temps, on a étudié la stabilité du film lacrymal en utilisant sur des lapins le test de LEMP (Arch. Ophtalm. Chicago 89 p.103, 105, 1973) qui mesure le temps de formation de zones sèches après

0 063 973

installation de fluorescéine de l'oeil. Si le temps de rupture du film est inférieur à 15 secondes le film est considéré comme instable alors qu'il est tenu pour stable lorsque ce temps est compris entre 15 et 25 secondes. On a pu obtenir, par analyse statistique des très nombreuses données récupérées, des résultats hautement significatifs qui ont toujours mis en évidence: une amélioration de l'humidification de l'oeil par le collyre selon l'invention, se traduisant par un film lacrymal plus long a se rompre pour l'oil traité; et également une bonne récupération de l'humidification de l'oeil.

Ces essais ont permis de conclure à la possibilité de tester le médicament en médecine humaine avec les indications: hypolacrymie due à une insuffisance de sécrétions lacrymales.

**h) Pharmacocinétique et disponibilité in vitro**

On s'est livré à des études permettant de savoir si le principe actif du collyre selon l'invention pouvait traverser la cornée.

Pour cela on a travaillé avec un appareillage approprié sur des cornées de bovins, maintenues en vie par perfusion et disposées dans un bac thermostaté. On administrait le collyre de l'invention sur la face épithéliale de la cornée et, après un délai de perfusion, on dosait la quantité de principe actif ayant pu traverser la cornée et se retrouvant donc du côté endothélial. Tout au long de la perfusion, l'intégralité de la cornée était contrôlée par un dispositif de mesure de la différence de potentiel produite par la cornée et par la mesure de l'épaisseur cornéenne à l'aide d'une lampe à fente. Les expériences ont été faites systématiquement d'une part sur le collyre de l'invention selon la composition précitée et, d'autre part, sur l'excipient seul de cette composition, à titre de témoin.

Malgré les conditions particulièrement favorables pour le passage de la chondroïtine sulfate A, il a été impossible de détecter ce produit dans le perfusat alors que des substances réputées pour leur faculté de traverser la cornée, telles que par exemple la procaïne et la pilocarpine, voyaient leurs quantités cumulées, après passage transcornéen, s'élever respectivement à 100 microgrammes et 200 microgrammes.

D'autres séries d'expérimentations de biodisponibilité orale chez le lapin, effectuées par technique de dosage des mucopolysaccharides totaux (taux endogène + chondroïtine sulfate A administrée) et par mesure de l'activité pharmacologique (clarification du plasma par l'administration d'héparine), ont permis de conclure que le principe actif selon l'invention n'était pas absorbé et qu'aucune toxicité systémique n'était donc à craindre pour le collyre à base de ce principe actif.

**i) Etudes cliniques sur sujets humains**

Les études cliniques, entreprises sur de nombreux malades en des séries de centres de soins différents, ont été faites en comparant l'action d'un collyre selon l'exemple précité de l'invention avec un collyre classique bien connu pour remplacer le larmoiement (sérum physiologique à 9‰ de chlorure de sodium). Dans tous les cas, la posologie d'emploi était de 1 à 2 gouttes dans chaque oeil malade, ceci 4 à 5 fois par jour pendant des périodes de temps déterminées (jusqu'à 6 mois). A chaque consultation des malades, on effectuait des séries de tests connus pour déterminer: la quantité des larmes, la qualité des larmes, la stabilité du film lacrymal et la tolérance (locale et générale).

Il ne paraît pas indispensable de fournir ici les résultats analytiques détaillés tenant compte des différents groupes de malades avec les paramètres: sexe, âge, durée de traitements (en moyenne 7 à 24 semaines). On se bornera donc à reproduire dans le tableau 1 ci-dessous les résultats statistiques globaux sur une série de malades:

**Tableau 1**

| traitements | résultats très bons | bons | modérés | nuls | mauvais | total |
|---|---|---|---|---|---|---|
| Collyre selon l'invention | 25 | 60 | 13 | 12 | 2 | 112 |
| Collyre de référence | 0 | 24 | 44 | 28 | 13 | 109[1]) |

[1]) certains essais ont été stoppés pour intolérance.

Ces résultats mettent bien en évidence la nette supériorité du collyre selon l'invention dans les deux séries de traitements comparés, puisque la proportion de très bons et bons résultats avec ce collyre est significativement plus élevée qu'avec le collyre témoin cependant bien connu pour son activité sur la sécheresse oculaire.

Il faut signaler par ailleurs que les maladies de l'oeil traitées par le collyre de l'invention étaient diverses et notamment de types: sécheresse lacrymale, kérato-conjonctive sèche, irritation conjonctivale, brûlure oculaire, larmoiement chronique des vieillards, syndromes de Gougerot-Sjögren, Riley-Day, polyarthrite évolutive chronique, maladie de Stevens-Johnson ... etc.

## Revendications

1. Collyre destiné aux affections de sécheresse oculaire, caractérisé en ce qu'il comporte comme seul principe actif le sel disodique du sulfate de chondroïtine A.

2. Collyre selon la revendication 1, caractérisé en ce qu'il renferme comme excipient au moins un: agent tampon, produit conservateur, agent isotonique, l'ensemble étant dissous dans de l'eau purifiée.

3. Collyre selon la revendication 2, caractérisé en ce que l'agent tampon est constitué par un mélange de phosphate dipotassique et de phosphate monopotassique.

4. Collyre selon l'une quelconque des revendications 2 ou 3, caractérisé en ce que le produit conservateur est constitué par du borate phénylmercurique.

5. Collyre selon l'une quelconque des revendications 2 à 4, caractérisé en ce que l'agent isotonique est de l'acide borique.

6. Composition aqueuse de collyre selon l'une quelconque des revendications 1 à 5, caractérisée en ce qu'elle comprend:

| | |
|---|---|
| Sel disodique du sulfate de chondroïtine | A 3 g |
| Phosphate dipotassique anhydre | 0,25 g |
| Phosphate monopotassique anhydre | 0,113 g |
| Acideborique | 1,2 g |
| Borate phénylmercurique | $\leqslant 0,0033$ g |
| Eau purifiée, quantité suffisante pour | 100 ml |

## Claims

1. Eye-lotion for the treatment of dry-eye diseases, characterized by the fact that it contains as single active component the disodic salt of chondroitine sulfate A.

2. Eye-lotion according the claim 1, wherein it contains as adjuvant at least a buffer agent, a preserving product, an isotonic agent, all these components being dissolved in purified water.

3. Eye-lotion according the claim 2, wherein the buffer agent is a mixture of monopotassic phosphate and dipotassic phosphate.

4. Eye-lotion according the claims 2 or 3, wherein the preserving product is the phenyl quicksilver borate.

5. Eye-lotion according anyone of the claims 2 to 4, wherein the isotonic agent is the boric acid.

6. Aqueous composition of the eye-lotion according anyone of the claims 1 to 5, characterized by the fact that it contains:

| | |
|---|---|
| di-sodic salt of chondroitine sulfate A | 3 g |
| anhydrous dipotassic phosphate | 0,25 g |
| anhydrous monopotassic phosphate | 0,113 g |
| boric acid | 1,2 g |
| phenylquicksilver borate | $\leqslant 0,0033$ g |
| purified water, sufficient quantity for | 100 ml |

## Patentansprüche

1. Augentropfen für die Behandlung des Syndroms des trockenen Auges, dadurch gekennzeichnet, dass es enthält als alleinigen aktiven Wirkstoff Chondroitinsulphat A in form von Di-Natrium Salz.

2. Augentropfen nach Anspruch 1, dadurch gekennzeichnet, dass sie als Adjuvans mindestens ein Puffer, ein Konservierungsmittel und eine isotonisierende Substanz enthalten, die in destilliertem Wasser aufgelöst sind.

3. Augentropfen nach Anspruch 2, dadurch gekennzeichnet, dass der Puffer aus einer Mischung von Di-Kalium Phosphat und Mono-Kalium Phosphat besteht.

4. Augentropfen nach Anspruch 2 oder 3, dadurch gekennzeichnet, dass· man als Konservierungsmittel Phenylquecksilberborat verwendet.

5. Augentropfen nach Ansprüchen 2, 3 oder 4, dadurch gekennzeichnet, dass man als isotonisierende Substanz Borsäure verwendet.

6. Augentropfen in form von wässriger Lösung, nach irgendeinem Anspruch 1 bis 5, dadurch gekennzeichnet, dass sie der folgenden Formulierung entsprechen

| | |
|---|---|
| Chondroitinsulphat A (Natriumsalz) | 3 g |
| Di-Kaliumphosphat (wasserfrei) | 0,25 g |
| Mono-Kaliumphosphat (wasserfrei) | 0,113 g |
| Borsäure | 1,2 g |
| Phenylquecksilberborat | $\leqslant$ 0,0033 g |
| Destilliertes Wasser...ad solut. pro | 100 ml |